# EUROPEAN PATENT APPLICATION

(11) **EP 1 264 830 A1**
(43) Date of publication of application: **11.12.2002**
(21) Application number: 01912204.3
(22) Date of filing: 08.03.2001
(51) Int. Cl.: C07D 341/00, C09K 3/00

(54) **CYCLIC ANILINE SULFIDES AND PROCESS FOR PREPARING THE SAME**

(30) Priority: 13.03.2000 JP 2000068566; 08.09.2000 JP 2000272991
(71) Applicant: COSMO OIL CO., LTD, Tokyo 105-0023 (JP)
(72) Inventor: MIYANO, Sotaro, Sendai-shi, Miyagi 982-0222 (JP); HATTORI, Tetsutaro, Sendai-shi, Miyagi 981-0952 (JP); IKI, Nobuhiko, 1-14, Kameoka Jutaku Daiichichiku, Sendai-shi, Miyagi 980-0865 (JP); MOROHASHI, Naoya, Sendai-shi, Miyagi 981-3108 (JP); KATAGIRI, Hiroshi, Sendai-shi, Miyagi 989-3205 (JP); TAKEYA, Haruhiko, c/o Cosmo Research Institute, Satte-shi, Saitama 340-0112 (JP); MIYANARI, Setsuko, c/o Cosmo Research Institute, Satte-shi, Saitama 340-0112 (JP); KOBORI, Toshihiro, c/o Cosmo Research Institute, Satte-shi, Saitama 340-0112 (JP)
(74) Representative: HOFFMANN - EITLE
(86) International application number: JP0101830
(87) International publication number: WO01068638

(57) **Abstract**

A cyclic phenol sulfide represented by the following formula: 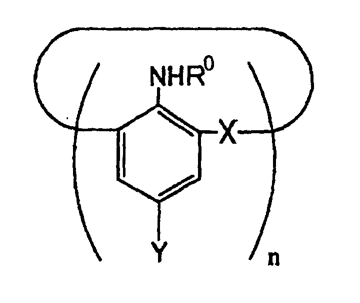 wherein X represents S, SO or SO₂; Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; R⁰ represents a hydrogen atom or a hydrocarbon group; plural X's or plural Y's are the same or different; and n represents an integer of 4 to 8, and a process for producing the same.

## Description

### TECHNICAL FIELD

The present invention relates to a cyclic aniline sulfide useful as a sensor, an analytical reagent, a raw material for metal-organic composite materials, and a metal scavenger, and a process for producing the cyclic aniline sulfide.

### BACKGROUND OF THE INVENTION

Recently, usefulness of crosslinked alkyl phenols having a cage structure has been variously reported as metal scavengers and substrate-specific sensors. Highly efficient processes for producing cyclic (cage) alkylphenol sulfides having a sulfur-crosslinked structure have hitherto been known (JP-A-9-227553 *etc.*), and the processes enable the utilization of the above functional substances and sulfonic acid derivatives thereof. However, these substances are intrinsically substances having an acidic group, and have no phenolic hydroxyl group and no residual group thereof. Furthermore, similar substances having a basic skeleton have not yet been obtained. Accordingly, use in an aqueous acidic solution is limited owing to the solubility and stability, and therefore it is desired to provide a derivative intrinsically having a basic skeleton, which can be utilized in a wide pH range and is applicable to an acidic medium.

### DISCLOSURE OF THE INVENTION

A problem to be solved by the present invention is to provide a cyclic aniline sulfide having a basic group usable even in a low pH range, for example, in an aqueous acidic solution, and a process for producing the same.

As a result of the extensive studies for achieving the above purpose, the present inventors have found an *N*-hydrocarbon group-substituted cyclic aniline sulfide obtainable by converting the hydroxyl group of a cyclic phenol sulfide having a sulfinyl group or a sulfonyl group, whose production method has already been laid open by the present inventors, into a hydrocarbon-oxy group and then reacting the product with a hydrocarbon group-substituted amino-lithium, and a process for producing the same, as well as a cyclic aniline sulfide obtainable by debenzylating the *N*-benzyl group-substituted cyclic aniline sulfide obtained therein and a process for producing the same. They have further found a cyclic aniline sulfide obtainable by reducing the sulfinyl group or sulfonyl group of the cyclic phenol sulfide having a sulfinyl group or a sulfonyl group, and a process for producing the same. Based on these findings, they have accomplished the present invention.

The present invention relates to the following (1) to (9):
(1) A cyclic aniline sulfide represented by formula (1): 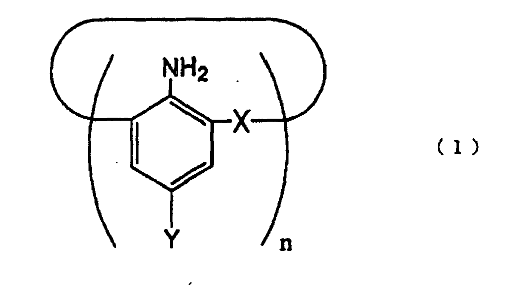 wherein X represents SO or SO₂; Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; plural X's or plural Y's are the same or different; and n represents an integer of 4 to 8.
(2) An *N*-hydrocarbon group-substituted cyclic aniline sulfide represented by formula (2): 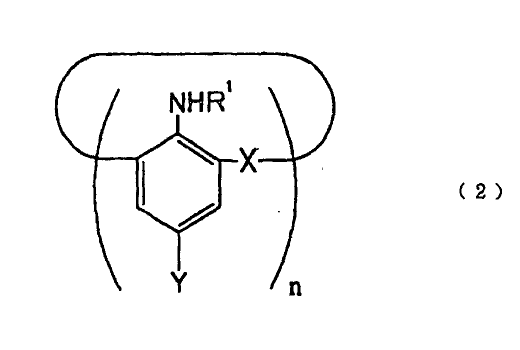 wherein X represents SO or SO₂; Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; R¹ is a hydrocarbon group; plural X's or plural Y's are the same or different; and n represents an integer of 4 to 8.
(3) A process for producing an *N*-hydrocarbon group-substituted cyclic aniline sulfide represented by formula (2): 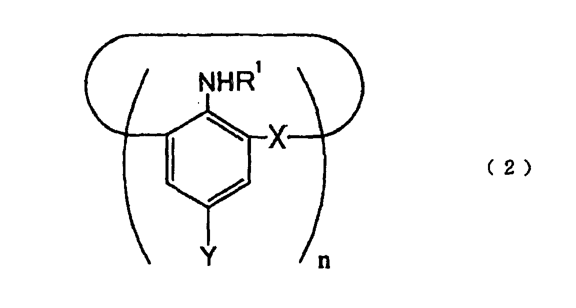 wherein X represents SO or SO₂; Y represents a hydrogen atom, a hydrocarbon group, a halogenated hydrocarbon group,
   -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; R¹ represents a hydrocarbon group; plural X's or plural Y's are the same or different; and n represents an integer of 4 to 8, which comprises reacting a cyclic phenol sulfide represented by formula (3): 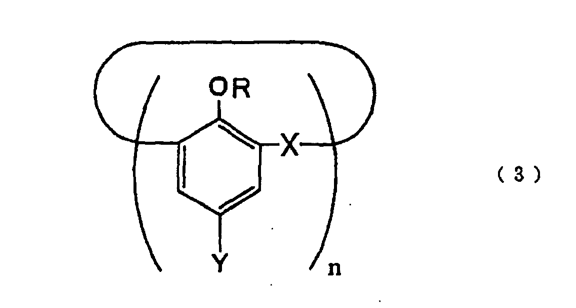 wherein X, Y, and n have the same meanings as described above; and R represents a hydrocarbon group, with a hydrocarbon group-substituted amino-lithium.
(4) The process for producing an *N*-hydrocarbon group-substituted cyclic aniline sulfide according to (3), wherein the hydrocarbon group-substituted amino-lithium is benzylamino-lithium.
(5) A process for producing a cyclic aniline sulfide represented by formula (1): 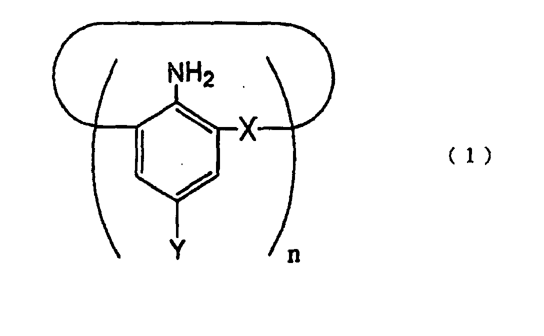 wherein X represents SO or SO₂; Y represents hydrogen atom,. a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; plural X's or plural Y's are the same or different; and n represents an integer of 4 to 8, which comprises debenzylating an *N*-benzyl group-substituted cyclic aniline sulfide represented by formula (2): 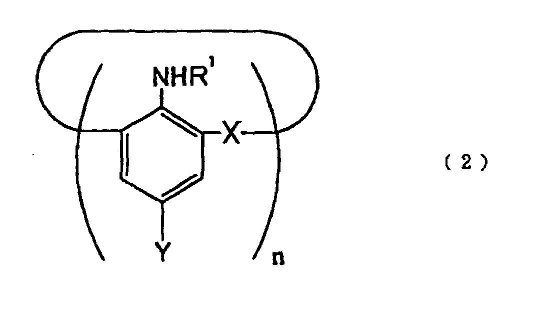 wherein X, Y and n have the same meanings as described above; and R¹ is a hydrocarbon group.
(6) The process for producing a cyclic aniline sulfide according to (5), wherein the debenzylation is conducted by substitution of a benzyl group with a halogen with a halogenating agent and hydrolysis with a basic catalyst.
(7) A cyclic aniline sulfide represented by formula (4): 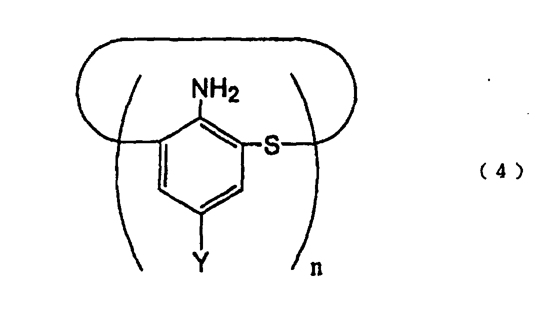 wherein Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; plural Y's are be the same or different; and n represents an integer of 4 to 8.
(8) A process for producing a cyclic aniline sulfide represented by formula (4): 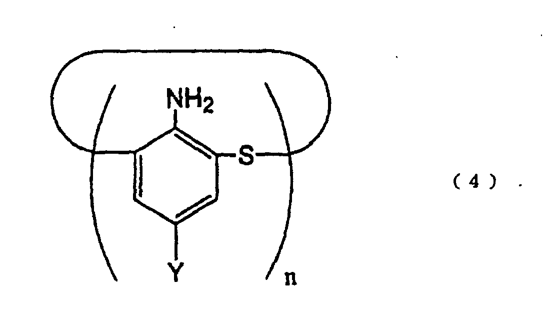 which comprises reducing a cyclic aniline sulfide represented by formula (1): 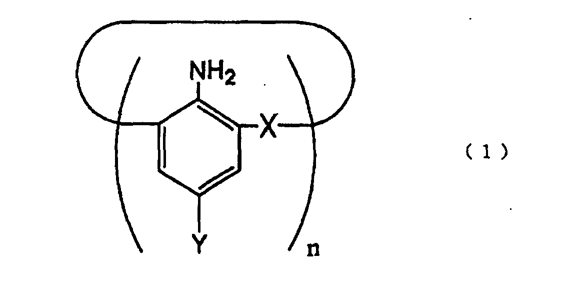 wherein Y and n have the same meanings as described above; X represents SO or SO₂; and plural X's are the same or different.
(9) The process for producing a cyclic aniline sulfide according to (8), wherein the reduction is conducted in the presence of titanium tetrachloride using lithium aluminum hydride.

### BEST MODE FOR CARRYING OUT THE INVENTION

In formula (1), X represents SO or SO₂, and plural X's are the same or different.

In formula (1), Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸, and plural Y's are the same or different.

Examples of the hydrocarbon group include a saturated aliphatic hydrocarbon group, an unsaturated aliphatic hydrocarbon group, an alicyclic hydrocarbon group, an alicyclic-aliphatic hydrocarbon group, an aromatic hydrocarbon group, an aromatic-aliphatic hydrocarbon group and the like.

Examples of the saturated aliphatic hydrocarbon group include an alkyl group such as methyl, ethyl, n-propyl, isopropyl, n-butyl, isobutyl, *tert*-butyl, n-pentyl, isopentyl, neopentyl, *tert*-pentyl, 2-methylbutyl, n-hexyl, isohexyl, 3-methylpentyl, ethylbutyl, n-heptyl, 2-methylhexyl, n-octyl, isooctyl, *tert*-octyl, 2-ethylhexyl, 3-methylheptyl, n-nonyl, isononyl, 1-methyloctyl, ethylheptyl, n-decyl, 1-methylnonyl, n-undecyl, 1,1-dimethylnonyl, n-dodecyl, n-tetradecyl, n-heptadecyl, n-octadecyl, *etc.;* a hydrocarbon group derived from a polymer or copolymer of ethylene, propylene or butylene; and the like.

Examples of the unsaturated aliphatic hydrocarbon group include alkenyl and alkynyl groups such as vinyl, allyl, isopropenyl, 2-butenyl, 2-methylallyl, 1,1,-dimetylallyl, 3-methyl-2-butenyl, 3-methyl-3-butenyl, 4-pentenyl, hexenyl, octenyl, nonenyl, decenyl, *etc.;* a hydrocarbon group derived from a polymer or copolymer of acetylene, butadiene or isoprene; and the like.

Examples of the alicyclic hydrocarbon group include cycloalkyl, cycloalkenyl and cycloalkynyl groups such as cyclopropyl, cyclobutyl, cyclopentyl, cyclohexyl, cycloheptyl, cyclooctyl, 3-methylcyclohexyl, 4-methylcyclohexyl, 4-ethylcyclohexyl, 2-methylcyclooctyl, cyclopropenyl, cyclobutenyl, cyclopentenyl, cyclohexenyl, cyclooctenyl, 4-methylcyclohexenyl, 4-ethylcyclohexenyl, *etc.*; and the like.

Examples of the alicyclic-aliphatic hydrocarbon group include alkyl, alkenyl and alkynyl groups substituted with a cycloalkyl, cycloalkenyl or cycloalkynyl group or the like, such as cyclopropylethyl, cyclobutylethyl, cyclopentylethyl, cyclohexylmethyl, cyclohexylethyl, cycloheptylmethyl, cyclooctylehtyl, 3-methylcyclohexylpropyl, 4-methylcyclohexylethyl, 4-ethylcyclohexylethyl, 2-methylcyclooctylethyl, cyclopropenylbutyl, cyclobutenylbylethyl, cyclopentenylethyl, cyclohexenylmethyl, cycloheptenylmethyl, cyclooctenylethyl, 4-methylcyclohexenylpropyl, 4-ethylcyclohexenylpentyl, *etc.*; and the like.

Examples of the aromatic hydrocarbon group include an aryl group such as phenyl, naphthyl, *etc.*; alkylaryl, alkenylaryl and alkynylaryl groups such as 4-methylphenyl, 3,4-dimethylphenyl, 3,4,5-trimethylphenyl, 2-ethylphenyl, n-butylphenyl, *tert*-butylphenyl, amylphenyl, hexylphenyl, nonylphenyl, 2-*tert*-butyl-5-methylphenyl, cyclohexylphenyl, cresyl, oxyethylcresyl, 2-methoxy-4-*tert*-butylphenyl, dodecylphenyl, *etc.*; and the like. The alkyl moiety of the alkylaryl group, the alkenyl moiety of the alkenylaryl group and the alkynyl moiety of the alkynylaryl group may be have a cyclic structure.

Examples of the aromatic-aliphatic hydrocarbon group include aralkyl, aralkenyl and aralykynyl groups such as benzyl, 1-phenylethyl, 2-phenylethyl, 2-phenylpropyl, 3-phenylpropyl, 4-phenylbutyl, 5-phenylpentyl, 6-phenylhexyl, 1-(4-methylphenyl)ethyl, 2-(4-methylphenyl)ethyl, 2-methylbenzyl, 1,1-dimethyl-2-phenylethyl, *etc.;* and the like. The alkyl moiety of the aralkyl group, the alkenyl moiety of the aralkenyl group and the alkynyl moiety of the aralykynyl group may be have a cyclic structure.

The halogen may be any of fluorine, chlorine, bromine and iodine.

Preferred examples of the halogenated hydrocarbon group include the above hydrocarbon groups substituted with at least one halogen.

R² to R⁸ each represents a hydrogen atom or a hydrocarbon group, and the structures of the above hydrocarbon groups are preferred.

The process for producing the cyclic aniline sulfide represented by formula (1) of the present invention is explained. The starting material is the cyclic phenol sulfide of formula (3).

The cyclic phenol sulfide represented by formula (3) is a known substance and Y in formula (3) is the same as Y in formula (1). R is a hydrocarbon group and specifically, a lower alkyl group such as methyl, ethyl, n-propyl, *tert*-butyl or the like is preferred. The process for producing the cyclic phenol sulfide represented by formula (3) is not particularly limited and the compound can be produced by optionally combining, for example, the method for cyclization through sulfuration and the method for modifying hydroxyl group described in JP-A-9-227553 and the sulfonylation method described in WO 98/09959.

The synthesis of the cyclic aniline sulfide of formula (1) is conducted in the order of conversion of the hydrocarbon-oxy group in the cyclic phenol sulfide of formula (3) into a hydrocarbon group-substituted amino group and removal of the hydrocarbon group, depending on the finally desired compounds.

First, the conversion of the hydrocarbon-oxy group into a hydrocarbon group-substituted amino group is explained.

The reaction is conducted by adding dropwise a hydrocarbon group-substituted amino-lithium prepared from a primary hydrocarbon-amine and a hydrocarbon-lithium to a cyclic phenol sulfide of formula (3), followed by stirring.

The molar ratio of the primary hydrocarbon-amine to the hydrocarbon-lithium is theoretically 1 : 1 but preferable results are usually obtained by use of a little excess hydrocarbon-lithium which is apt to decompose. As an actual mixing ratio (molar ratio), the reaction is operable in the range that the molar ratio of the primary hydrocarbon-amine to the hydrocarbon-lithium is from 1 : 1 to 1 : 2, preferably from 1 : 1 to 1 : 1.5, more preferably from 1 : 1 to 1 : 1.2.

The primary hydrocarbon-amine includes methylamine, ethylamine, n-propylamine, n-butylamine, n-pentylamine, n-hexylamine, benzylamine, phenethylamine, and the like, depending on the final synthetic purposes. When dealkylation is finally conducted, benzylamine is preferred. The hydrocarbon-lithium includes methyllithium, ethyllithium, n-propyllithium, n-butyllithium, n-pentyllithium, n-hexyllithium, benzyllithium and the like, but n-butyllithium is preferred in view of the reactivity.

The hydrocarbon group-substituted amino-lithium is preferably prepared in the presence of a solvent. The solvent includes ether solvents such as dimethyl ether, diethyl ether, tetrahydrofuran (THF), and dioxane, acetonitrile and the like. As the amount of the solvent to be used, the reaction is operable in the range of 0.5 to 100 ml per mmol of the amine, and preferred is from 1 to 10 ml per mmol of the amine in view of solvent cost and reaction efficiency. The reaction is preferably conducted under an inert gas atmosphere at a low temperature, and can be conducted under an atmosphere of dry nitrogen, argon, or the like at 0°C or lower, preferably at -30°C or lower. The reaction may finish at the same time when the addition is completed, but it is preferred to stir the mixture at -15°C to 0°C for 1 to 3 hours after the addition in order to complete the reaction.

In the reaction between the hydrocarbon group-substituted amino-lithium thus prepared and the cyclic phenol sulfide of formula (3), the theoretical mixing ratio is equimolar amount of the hydrocarbon group-substituted amino-lithium to the number of the hydrocarbon-oxy group in the cyclic phenol sulfide of formula (3). Actually, the reaction is operable in the range that the molar ratio of the hydrocarbon-oxy group to the hydrocarbon group-substituted amino-lithium is from 1 : 1 to 1 : 2, but a preferred range is from 1 : 1.1 to 1 : 1.5 in view of the cost of the hydrocarbon group-substituted amino-lithium and the like.

The reaction is preferably conducted in the presence of a solvent. The solvent includes ether solvents such as dimethyl ether, diethyl ether, tetrahydrofuran (THF), and dioxane, acetonitrile, and the like. As the amount of the solvent to be used, the reaction is operable in the range of 1 to 500 ml per mmol of the cyclic phenol sulfide of formula (3), and preferred is from 10 to 100 ml per mmol of the cyclic phenol sulfide of formula (3) in view of solvent cost and reaction efficiency. The reaction is preferably conducted under an inert gas atmosphere at a low temperature, and is preferably conducted under an atmosphere of dry nitrogen, argon, or the like at 10°C or lower, preferably at 0°C or lower. However, since the reaction rate decreases as the reaction proceeds, it is preferred to elevate the temperature to 10 to 20°C over 1 to 3 hours after the addition.

After completion of the reaction, the product may be purified according to a usual manner, and a desired *N*-benzyl group-substituted cyclic aniline sulfide of formula (2) is usually obtained by deactivating the unreacted reactant with an aqueous ammonium chloride solution, followed by stirring after addition of dichloromethane, chloroform or the like, separating the organic layer, washing it with water, and drying the layer over anhydrous magnesium sulfate, finally followed by filtration and concentration.

Next, debenzylation of the *N*-benzyl group-substituted cyclic aniline sulfide is explained.

In the case that the hydrocarbon group in the *N*-hydrocarbon group-substituted cyclic aniline sulfide is benzyl group, amino group may be formed by halogen-substitution of the benzyl group with a halogenating agent and successive hydrolysis with a basic catalyst.

The halogenating agent for use in the halogen-substitution includes iodine, bromine, chlorine, *etc.*, and *N*-halogenated succinimide and the like. Preferred are bromine and *N*-bromosuccinimide in consideration of the balance of convenience in operation and reactivity. As the amount of the halogenating agent to be used, a theoretical mixing ratio is use of equimolar amount relative to the number of the benzyl group in the *N*-benzyl group-substituted cyclic aniline sulfide of formula (2). Actually, the reaction is operable in the range that the molar ratio of the benzyl group to the halogenating agent is from 1 : 1 to 1 : 2, but preferred range is from 1 : 1.1 to 1 : 1.5 in view of the cost of the halogenating agent and the like.

The reaction is preferably conducted under a radical atmosphere. Specifically, it is preferred to initiate the reaction under irradiation with ultraviolet ray or a ray containing the same, or to employ a catalytic amount of a radical initiator such as benzoyl peroxide, azobisisobutyronitrile or the like.

The reaction is preferably conducted in the co-presence of a solvent, and use may be made of a halogenated solvent such as dichloromethane, chloroform, carbon tetrachloride or the like, or an aromatic hydrocarbon such as benzene or the like. As the amount of the solvent to be used, the reaction is operable in the range of 10 to 500 ml per mmol of the *N*-benzyl group-substituted cyclic aniline sulfide of formula (2), and preferred is from 20 to 100 ml per mmol of the *N*-benzyl group-substituted cyclic aniline sulfide of formula (2) in view of solvent cost and reaction efficiency.

The reaction temperature at that time is preferably from 0 to 150°C, and good reaction results are usually obtained at a temperature of 20°C, i.e., room temperature to 100°C. The reaction time varies depending on the reaction temperature, but it may be from 15 minutes to 3 hours at a reaction temperature of 20 to 100°C.

The *N*-α-halobenzylated cyclic aniline sulfide thus obtained is dehalobenzylated by hydrolysis in the presence of a basic catalyst to form a cyclic aniline sulfide of formula (1) having no substituent on the nitrogen atom. As the basic catalyst, for example, KOH, NaOH, CsOH or the like may be optionally used. The basic catalyst is preferably added as an aqueous solution and preferred concentration range is from 0.1 M to 5 M. Moreover, the molar ratio of the basic catalyst to be used relative to the number of the halogen in the *N*-halogenated cyclic aniline sulfide is theoretically 1 : 1, but the reaction is usually operable in the range of 1 : 1 to 1 : 2, preferably 1 : 1 to 1 : 1.5, more preferably 1 : 1 to 1 : 1.2.

For the purpose of enhancing the reaction efficiency, the debenzylation is preferably conducted in the co-presence of a solvent, and an ether solvent such as diethyl ether, THF, dioxane or the like may be used. Moreover, hexamethylphosphoric triamide or the like may be co-existed in order to dissolve the basic catalyst. As the amount of the solvent to be used, the reaction is operable in the range of 10 to 500 ml per mmol of the *N*-halogenated cyclic aniline sulfide, and preferred is from 20 to 100 ml per mmol of the *N*-halogenated cyclic aniline sulfide in view of solvent cost and reaction efficiency. The reaction temperature at that time is preferably from 0 to 150°C, and good reaction results are usually obtained at a temperature of 20 to 100°C. The reaction time varies depending on the reaction temperature, but it may be usually from 6 hours to 3 days at a reaction temperature of 20 to 100°C. The obtained cyclic aniline sulfide of formula (1) may be preferably subjected to a usual purification such as recrystallization.

Next, the cyclic aniline sulfide of formula (4) will be explained.

Y in formula (4) is a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸, and plural Y's are the same or different.

Preferred examples of Y include those described as Y in formula (1). Moreover, with regard to the hydrocarbon group and halogen atom, those described in formula (1) are exemplified.

R² to R⁸ each represents a hydrogen atom or a hydrocarbon group, and as preferable examples, the skeleton described as the above hydrocarbon group may be applicable.

The process for producing a cyclic aniline sulfide represented by formula (4) of the present invention is explained below. The starting material is a cyclic aniline sulfide of formula (1), and preferred is a compound wherein X is a sulfinyl group. As the reduction of the sulfonyl group or sulfinyl group in the starting cyclic aniline sulfide of formula (1), the cyclic aniline sulfide of formula (4) is provided. The method for the reduction is not limited, but the cyclic aniline sulfide of formula (4) can be obtained in high yields by treating the starting material with lithium aluminum hydride in the presence of titanium tetrachloride. The reaction is conducted by adding dropwise lithium aluminum hydride and titanium tetrachloride successively to the starting material dissolved in a solvent. It is theoretically sufficient that the molar ratio of the starting material to lithium aluminum hydride is 1 : 1, but more preferred results are obtained by use of excess lithium aluminum hydride which is apt to decompose during the operation. Actually, preferable results are obtained by use of lithium aluminum hydride in an amount of 5 to 100 mol, preferably 10 to 40 mol, per mol of the starting material. The amount of titanium tetrachloride to be used is preferably from 0.3 to 1.5 mol, more preferably 0.4 to 1 mol, per mol of lithium aluminum hydride.

As the solvent, ether solvents such as diethyl ether, THF, dioxane, *etc.,* acetonitrile, DMF and the like may be used. As the amount of the solvent to be used, the reaction is operable in the range of 10 to 200 ml per mmol of the starting material, and preferred is from 50 to 150 ml in view of solvent cost and reaction efficiency. The reaction is preferably conducted under inert gas atmosphere at a low temperature. The reaction is operable under atmosphere of dry nitrogen, argon or the like at 30°C or lower, preferably 20°C or lower, but the above dropwise addition of lithium aluminum hydride and titanium tetrachloride is preferably conducted at -40°C or lower, more preferably -70°C or lower. Most of the reaction finishes at the same time when the addition is completed, but it is preferred to elevate the temperature to 10 to 20°C over 1 to 3 hours after the addition since the reaction rate decreases as the reaction proceeds.

After completion of the reaction, the product may be purified according to a usual manner. Usually, an aimed cyclic aniline sulfide of formula (4) is obtained by deactivating unreacted reactants with an aqueous ammonium chloride solution, acidifying the solution with dilute hydrochloric acid, followed by stirring after the addition of dichloromethane, chloroform or the like, separating the organic layer, washing it with water, and drying the layer over anhydrous magnesium sulfate, finally followed by filtration and concentration.

The present invention is described below in further detail with reference to Production Examples, Examples, and Application Examples, but the present invention is not limited to these Examples.

### Production Example 1

### Synthesis of 5,11,17,23-tetra-tert-butyl-25,26,27,28-tetrahydroxy-2,8,14,20-tetrathia[1.9.3.1.1^{3,7}1^{9,13}1^{15,19}]-octacosa-1(25),3,5,7(28),9,11,13(27),15,17,19(26),21,23dodecaene (I):

To 45.2 g of 4-*tert*-butylphenol were added 14.4 g elemental sulfur, 3.0 g of sodium hydroxide and 7.60 g of tetraethylene glycol, followed by gradually heating up to 230°C over 4 hours under stirring in an atmosphere of nitrogen, and followed by stirring for further 2 hours. During the reaction, water and a hydrogen sulfide generated by the reaction were removed. The reaction mixture was cooled to room temperature, dissolved in 500 ml of ether added, and hydrolyzed with 1 N aqueous sulfuric acid solution. A separated ether layer was washed with water and dried with magnesium sulfate. The reaction mixture obtained by evaporation of ether was further fractionized by silica gel column chromatography (hexane/chloroform) to thereby obtain a crude product. The crude product was recrystallized from chloroform/acetone to thereby obtain 26.5 g of 5,11,17,23-tetra-*tert*-butyl-25,26,27,28-tetrahydroxy-2,8,14,20-tetrathia[1.9.3.1.1^{3,7}1^{9,13}1^{15,19}]octacosa-1(25),3,5,7(28),9,11,13(27),15,17,19(26),21,23-dodecaene as colorless transparent crystals. The yield was 45%.

### Production Example 2

### Synthesis of 5,11,17,23-tetra-tert-butyl-25,26,27,28-tetramethoxy-2,8,14,20-tetrathia[1.9.3.1.1^{3,7}1^{9,13}1^{15,19}]octacosa-1(25),3,5,7(28),9,11,13(27),15,17,19(26),21,23-dodecaene.

Into a flask made of glass was placed 9.28 g (12.87 mmol) of 5,11,17,23-tetra-*tert*-butyl-25,26,27,28-tetrahydroxy-2,8,14,20-tetrathia[1.9.3.1.1^{3,7}1^{9,13}1^{15,19}]octacosa-1(25),3,5,7(28),9,11,13(27),15,17,19(26),21,23-dodecaene obtained in Production Example 1 together with 50 ml of acetone, and the mixture was stirred together with 5.34 g (38.61 mmol) of anhydrous potassium carbonate and suspended. Thereto was added 8.77 g (61.78 mmol) of methyl iodide at room temperature (about 20°C) over 1 hour, followed by heating under reflux for 24 hours. After completion of the reaction, the reaction mixture was cooled to room temperature and, after removal of a precipitate by filtration, the filtrate was concentrated and dried. The residue was washed with methanol to thereby obtain 9.01 g (11.59 mmol) of 5,11,17,23-tetra-*tert*-butyl-25,26,27,28-tetramethoxy-2,8,14,20-tetrathia[1.9.3.1.1^{3,7}1^{9,13}1^{15,19}]octacosa-1(25),3,5,7(28),9,11,13(27),15,17,19(26),21,23-dodecaene as a white powder. The yield was 90%.

### Production Example 3

### Synthesis of 5,11,17,23-tetra-tert-butyl-25,26,27,28-tetramethoxy-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene.

Into a flask made of glass was placed 5.00 g (6.43 mmol) of 5,11,17,23-tetra-*tert*-butyl-25,26,27,28-tetramethoxy-2,8,14,20-tetrathia[1.9.3.1.1^{3,7}1^{9,13}1^{15,19}]octacosa-1(25),3,5,7(28),9,11,13(27),15,17,19(26),21,23-dodecaene together with 50 ml of dichloromethane, and the mixture was stirred together with 0.733 g (6.43 mmol) of trifluoroacetic acid and dissolved. Thereto was added 5.10 g (60.00 mmol) of 40% aqueous hydrogen peroxide solution at room temperature (about 20°C) over 1 hour, followed by heating under reflux for 24 hours. After completion of the reaction, the reaction mixture was cooled to room temperature. Water-soluble components were washed and removed using a separating funnel. Thereafter, the mixture was concentrated and dried and the residue was recrystallized from dichloromethane-hexane to thereby obtain 4.89 g (5.40 mmol) of 5,11,17,23-tetra-*tert*-butyl-25,26,27,28-tetramethoxy-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene as a white powder. The yield was 84%.

### Production Example 4

### Synthesis of 5,11,17,23-tetra-tert-butyl-25,26,27,28-tetrahydroxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene.

Into 300 ml of chloroform was dissolved 18 g of 5,11,17,23-tetra-*tert*-butyl-25,26,27,28-tetrahydroxy-2,8,14,20-tetrathia[1.9.3.1.1^{3,7}1^{9,13}1^{15,19}]octacosa-1(25),3,5,7(28),9,11,13(27),15,17,19(26),21,23-dodecaene obtained in Production Example 1. A solution of 57 g of 30% aqueous hydrogen peroxide dissolved in 1000 ml of glacial acetic acid beforehand was added dropwise to the chloroform solution over 60 minutes, followed by stirring at room temperature for further 24 hours. To the resulting reaction solution was added 1500 ml of water, and extraction with chloroform (500 ml × 3) was conducted and the chloroform layer was washed with water. The chloroform solution was dried over anhydrous magnesium sulfate, the solvent was removed by evaporation, and 5.2 g of the resulting white powder was thoroughly washed with methanol to thereby obtain 4.85 g of a product.

The product is a cyclic phenol sulfide wherein X is SO, Y is *tert*-butyl, and n is 4 in formula (3).

The results of the physical properties are shown below.
Melding point: 210°C (decomposed)
¹H-NMR: (500 MHz; CDCl₂CDCl₂): δ (ppm) 9.20 (s, 4H, OH), 7.61 (s, 8H, ArH), 1.26 (s, 36H, C(CH₃)₃)
¹³C-NMR: (500 MHz; CDCl₂CDCl₂): δ (ppm) 152.7, 142.4, 130.2, 128.0, 124.2, 122.8 (Ar), 34.8 (C(CH₃)₃), 31.4 (C(CH₃)₃)
FT-IR: (cm⁻¹, KBr): 3074 (OH), 2960 (CH), 1051, 998 (SO)
MS (m/z): 785 (M⁺+1)

| Elemental analysis (%) | |
|---|---|
| Calculated for C₄₀H₄₈S₄O₄ | C: 61.20, H: 6.16, S: 16.34 |
| Found | C: 61.1, H: 6.3, S: 15.9 |

### Production Example 5

### Synthesis of 5,11,17,23-tetra-tert-butyl-25,26,27,28-tetramethoxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene

In a flask made of glass under nitrogen atmosphere was placed 6.09 g (7.76 mmol) of 5,11,17,23-tetra-*tert*-butyl-25,26,27,28-tetrahydroxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene obtained in Production Example 4 together with 140 ml of anhydrous acetone, and then 17.4 g (126 mmol) of anhydrous potassium carbonate and 9.0 ml (144 mmol) of methyl iodide were added thereto. The mixture was heated under reflux to be allowed to react for 3 days. The reaction mixture was cooled to 0°C, and then the reaction was terminated by adding 2 N hydrochloric acid. From the mixed solution, a reaction product was extracted three times by liquid-liquid extraction using chloroform. The extract solution was concentrated and dried and the resulting crude product was recrystallized from dichloromethane-n-hexane to thereby obtain 5.66 g of 5,11,17,23-tetra-*tert*-butyl-25,26,27,28-tetramethoxy-2,8,14,20-tetrasulfinyl[1.9.3.1.1^{3,7}1^{9,13}1^{15,19}]-octacosa-1(25),3,5,7(28),9,11,13(27),15,17,19(26),21,23-dodecaene as a white powder (yield 87%).

The results of the physical properties are shown below.
¹H-NMR (500 MHz; CDCl₃): δ (ppm) 1.32 (s, 36H, C(CH₃)₃), 3.92 (s, 12H, OCH₃), 7.68 (d, 4H, J = 2.4 Hz, ArH), 8.13 (d, 4H, J = 2.4, ArH)
IR (KBr): cm⁻¹ 2964 (CH), 1269 (COC), 1055 (SO), 991 (COC)

| Elemental analysis (%) | |
|---|---|
| Calculated for C₄₄H₅₆O₈S₄ | C: 62.83, H: 6.71, S: 15.25 |
| Found | C: 62.64, H: 6.68, S: 15.50 |

### Example 1

### Synthesis of 5,11,17,23-tetra-tert-butyl-25,26,27,28-tetrabenzylamino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene

In a 200 ml three-necked round-bottom flask replaced by nitrogen was placed 75 ml of anhydrous tetrahydrofuran (THF) and 2.83 g (26.4 mmol) of benzylamine, followed by cooling to -78°C in a dry ice-methanol bath. Thereto was added dropwise 19.8 ml (31.7 mmol) of 1.6 M hexane solution of n-butyllithium over 1 hour, and, after completion of the addition, followed by stirring at 0°C for 1 hour (Liquid A).

In a 300 ml three-necked round-bottom flask was placed 6.10 g (5.52 mmol) of 5,11,17,23-tetra-*tert*-butyl-25,26,27,28-tetramethoxy-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene. After replacement by nitrogen, 90 ml of anhydrous tetrahydrofuran was added thereto and the white-turbid solution was cooled to 0°C. Liquid A was added dropwise thereto over 1 hour and, after completion of the addition, the mixture was stirred at room temperature for 2 hours. After 2 hours, under cooling the flask on a water bath, the reaction was terminated by adding 10 ml of a saturated aqueous NH₄Cl solution, 2 N HCl was added thereto and then the mixture was extracted with 50 ml of chloroform three times. The extract solution was washed twice with distilled water and the solvent was removed by evaporation to thereby obtain 7.02 g of a pale yellow crude product. The product was washed with acetone and dissolved in methylene chloride, which was filtered while hot. Thereafter, the filtrate was recrystallized (CHCl₃-EtOH) to thereby obtain 4.66 g (3.87 mmol) of 5,11,17,23-tetra-*tert*-butyl-25,26,27,28-tetrabenzylamino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene as a white powder. The yield was 70%.

The results of the measured physical properties of the cyclic aniline sulfide obtained in Example 1 (tetrabenzylamino-sulfonyl compound) are shown below.
¹H-NMR (500 MHz; CDCl₃): δ (ppm): 0.88 (s, 36H, C(CH₃)₃), 2.49 (t, 4H, J = 7.1 Hz, ArNHCH₂Ar), 4.41 (d, 8H, J= 7.1 Hz, ArNHCH₂Ar), 7.27-7.35 (m, 12H, ArNHCH₂ArH), 7.56-7.57 (m, 8H, ArNHCH₂ArH), 8.35 (s, 8H, ArHNHCH₂Ar)
IR (KBr): cm⁻¹ 3350 (NH), 2964 (CH), 1321 (SO₂), 1155 (SO₂)
FAB MS m/z 1205 (M⁺+1)
Melting point: >360°C,

| Elemental analysis (%) | |
|---|---|
| Calculated for C₆₈H₇₆N₄O₈S₄ | C: 67.74, H: 6.35, N: 4.65, S: 10.64 |
| Found | C: 67.78, H: 6.35, N: 4.56, S: 10.75 |

### Example 2

### Synthesis of 5,11,17,23-tetra-tert-butyl-25,26,27,28-tetraamino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene

In a 300 ml three-necked round-bottom flask were placed 3.50 g (2.90 mmol) of 5,11,17,23-tetra-*tert*-butyl-25,26,27,28-tetrabenzylamino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene obtained in Example 1 and 2.27 g (12.76 mmol) of *N*-bromosuccinimide and, after replacement by nitrogen, 90 ml of anhydrous benzene was added thereto. Thereto was added 1 mg of benzoyl peroxide, and the mixture was heated and stirred under benzene reflux for 1 hour. Then, the reaction mixture was cooled to room temperature, a formed white precipitate (succinimide) was removed by filtration, and the filtrate was placed in a separating funnel, washed once with water, and then concentrated and dried to thereby obtain a white powder. The powder was recrystallized from tetrahydrofuran to thereby obtain 3.20 g (2.49 mmol) of 5,11,17,23-tetra-*tert*-butyl-25,26,27,28-tetra(.-bromobenzyl)amino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene. The yield was 86%.

In a 300 ml three-necked round-bottom flask was placed 2.50 g (1.95 mmol) of 5,11,17,23-tetra-*tert*-butyl-25,26,27,28-tetra(.-bromobenzyl)amino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene, and 60 ml of THF and 30 ml of hexamethylphosphoric triamide were added thereto. Thereto was added 8 ml of a 1.0 M aqueous KOH solution, and the mixture was heated and stirred at 70°C for 48 hours. Then, the reaction mixture was cooled to room temperature and a formed precipitate was recovered by filtration, which was recrystallized from THF to thereby obtain 1.30 g (1.54 mmol) of 5,11,17,23-tetra-*tert*-butyl-25,26,27,28-tetraamino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene. The yield was 79%.

The results of the measured physical properties of the cyclic aniline sulfide obtained in Example 2 (tetraamino-sulfonyl compound) are shown below.
¹H-NMR (500 MHz; DMSO-d₆): δ (ppm) 1.24 (s, 36H, C(CH₃)₃), 5.65 (s, 8H, NH), 7.99 (s, 8H, ArH)
IR (KBr): cm⁻¹ 3468 (NH), 3389 (NH), 2964 (CH), 1313 (SO₂), 1151 (SO₂)
FAB MS m/z 845 (M⁺+1)
Melting point: >360°C

| Elemental analysis (%) | |
|---|---|
| Calculated for C₄₀H₅₂N₄O₈S₄ | C: 57.08, H: 6.38, N: 6.03, S: 14.65 |
| Found | C: 56.85, H: 6.20, N: 6.63, S: 15.18 |

### Example 3

### Synthesis of 5,11,17,23-tetra-tert-butyl-25,26,27,28-tetrabenzylamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene

In a 200 ml three-necked round-bottom flask replaced by nitrogen was placed 70 ml of anhydrous THF and 12.0 g (110 mmol) of benzylamine, and the mixture was cooled to -78°C in a dry ice-methanol bath. Thereto was added dropwise 56.3 ml (88.3 mmol) of a 1.6 M hexane solution of n-butyllithium over 1 hour, and, after completion of the addition, the mixture was stirred at 0°C for 1 hour (Liquid A).

In a 300 ml three-necked round-bottom flask was placed 5.28 g (6.28 mmol) of 5,11,17,23-tetra-*tert*-butyl-25,26,27,28-tetramethoxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene. After replacement by nitrogen, 90 ml of anhydrous THF was added thereto and the white-turbid solution was cooled to 0°C. Liquid A was added dropwise thereto over 1 hour and, after completion of the addition, the mixture was stirred at room temperature for 2 hours. After 2 hours, under cooling the flask on a water bath, the reaction was terminated by adding 10 ml of a saturated aqueous NH₄Cl solution, 2 N HCl was added thereto, and the mixture was extracted with 50 ml of chloroform three times. The extract solution was washed twice with distilled water and the solvent was removed by evaporation to thereby obtain a white crude product. The product was washed with acetone and dissolved in methylene chloride, and the solution was filtered while hot. Thereafter, the filtrate was recrystallized (CHCl₃-EtOH) to thereby obtain 4.56 g (4.02 mmol) of 5,11,17,23-tetra-*tert*-butyl-25,26,27,28-tetrabenzylamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene as a white powder (yield 64%).

The results of the measured physical properties of the obtained cyclic aniline sulfide (tetrabenzylaminosulfinyl compound) are shown below.
¹H-NMR (500 MHz; CDCl₃): δ (ppm) 0.90 (s, 36H, C(CH₃)₃), 3.34 (dd, 4H, J = 11.1 Hz, 2.9 Hz, ArNHCH₂Ar), 4.17 (t, 4H, J= 11.1, ArNHCH₂Ar), 4.27 (dd, 4H, J = 11.1 Hz, 2.9 Hz, ArNHCH₂Ar), 7.30 (tt, 4H, J = 7.4, 1.8 Hz, ArH), 7.37 (t, 8H, J = 7.4 Hz, ArH), 7.59 (d, 8H, J = 7.4 Hz, ArH), 7.60 (d, 4H, J = 2.4 Hz, ArH), 7.92 (d, 4H., J = 2.4 Hz, ArH)
IR (KBr): cm⁻¹ 3354 (NH), 2963 (CH), 1045 (SO)

| Elemental analysis (%) | |
|---|---|
| Calculated for C₆₈H₇₆N₄O₄S₄ | C: 71.56, H: 6.71, N: 4.91, S: 11.24 |
| Found | C: 71.30, H: 6.75, N: 4.79, S: 11.18 |

### Example 4

### Synthesis of 5,11,17,23-tetra-tert-butyl-25,26,27,28-tetrabenzylidenamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene

In a 500 ml of three-necked round-bottom flask were placed 4.39 g (3.85 mmol) of 5,11,17,23-tetra-*tert*-butyl-25,26,27,28-tetrabenzylamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene obtained in Example 3 and 4.23 g (23.8 mmol) of *N*-bromosuccinimide. After replacement by nitrogen, 250 ml of anhydrous benzene was added thereto. Thereto was added 411 mg of benzoyl peroxide, and the mixture was heated and stirred under benzene reflux for 1 hour. The reaction mixture was cooled to room temperature and 5 wt% aqueous sodium hydrogen sulfite solution was added thereto, followed by stirring. Then, the reaction mixture was cooled to room temperature, a formed white precipitate (succinimide) was removed by filtration, and the filtrate was concentrated and dried. Then, the residue was washed only a little amount of methanol to thereby obtain a white powder. The powder was recrystallized from dichloromethane-methanol to thereby obtain 3.69 g (3.25 mmol) of 5,11,17,23-tetra-*tert*-butyl-25,26,27,28-tetrabenzylidenamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene (yield 85%).

The results of the measured physical properties of the obtained cyclic aniline sulfide (tetrabenzylideaminosulfinyl compound) are shown below.
¹H-NMR (400 MHz; CDCl₃) : δ (ppm) 0.86 (s, 36H, C(CH₃)₃), 7.46 (t, 8H, J = 13.6, ArH), 7.46 (d, 8H, J = 13.6 Hz, ArH), 7.55-7.59 (m, 12H, ArH), 8.42 (s, 4H, ArNCHAr)
IR (KBr): cm⁻¹ 2955 (CH), 1631 (NC), 1047 (SO)

| Elemental analysis (%) | |
|---|---|
| Calculated for C₆₈H₆₈N₄O₄S₄ | C: 72.05, H: 6.05, N: 4.94, S: 11.32 |
| Found | C: 71.68, H: 6.10, N: 4.84, S: 11.10 |

### Example 5

In a 500 ml three-necked round-bottom flask was placed 3.07 g (2.71 mmol) of 5,11,17,23-tetra-*tert*-butyl-25,26,27,28-tetrabenzylidenamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene obtained in Example 4, and 200 ml of chloroform and 100 ml of concentrated hydrochloric acid were added thereto, followed by heating under reflux for 12 hours. Then, the reaction mixture was cooled to room temperature and the product was extracted with chloroform three times. The extract was concentrated and dried, and then the residue was washed with methanol and dried under reduced pressure to thereby obtain a white solid. The white solid was recrystallized from a mixed solution of dichloromethane-methanol to thereby obtain 1.80 g (2.30 mmol) of 5,11,17,23-tetra-*tert*-butyl-25,26,27,28-tetraamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene as white crystals (yield 85%).

The results of the measured physical properties of the obtained cyclic aniline sulfide (tetraamino-sulfinyl compound) are shown below.
¹H-NMR (400 MHz; DMSO-d₆, 110°C): δ (ppm) 1.17 (s, 36H, C(CH₃)₃), 5.36 (s, 8H, NH₂), 7.56 (s, 8H, ArH)
IR (KBr): cm⁻¹ 3452 (NH), 3371 (NH), 2959 (CH), 1030 (SO)

| Elemental analysis (%) | |
|---|---|
| Calculated for C₄₀H₅₂N₄O₄S₄ | C: 61.50, H: 6.71, N: 7.17, S: 16.42 |
| Measured | C: 61.43, H: 6.61, N: 7.11, S: 16.69 |

### Example 6

### Synthesis of 5,11,17,23-tetra-tert-butyl-25,26,27,28-tetraamino-2,8,14,20-tetrathia[1.9.3.1.1^{3,7}1^{9,13}1^{15,19}]octacosa-1(25),3,5,7(28),9,11,13(27),15,17,19(26),21,23-dodecaene

In a 300 ml three-necked round-bottom flask was placed 1.58 g (2.02 mmol) of 5,11,17,23-tetra-*tert*-butyl-25,26,27,28-tetraamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene obtained in Example 5. After replacement by nitrogen, 160 ml of THF and 1.23 g (32.4 mmol) of lithium aluminum hydride were added thereto, followed by cooling to -78°C. Thereto was added dropwise 1.7 ml (15.47 mmol) of titanium chloride, and the whole was stirred at -78°C for 15 minutes, followed by stirring at room temperature for 2 hours. Then, a saturated aqueous ammonium chloride solution was added to the reaction mixture under ice cooling, and then 20 ml of 2 N hydrochloric acid was added thereto. The mixture was. extracted with chloroform three times from the resulting aqueous acidic solution. The chloroform solution was concentrated and dried, and the resulting solid was washed with methanol and dried under reduced pressure to thereby obtain a white crude product. The crude product was recrystallized from a mixed solution of dichloromethane-methanol to thereby obtain 940 mg (1.31 mmol) of 5,11,17,23-tetra-*tert*-butyl-25,26,27,28-tetraamino-2,8,14,20-tetrathia[1.9.3.1.1^{3,7}1^{9,13}1^{15,19}]octacosa-1(25),3,5,7(28),9,11,13(27),15,17,19(26),21,23-dodecaene as white crystals (yield: 65%).

The results of the measured physical properties of the obtained cyclic aniline sulfide (tetraamino-sulfide compound) are shown below.
¹H-NMR (400 MHz; CDCl₃): δ (ppm) 1.12 (s, 36H, C(CH₃)₃), 4.88 (br, 8H, NH₂), 7.35 (s, 8H, ArH)
IR (KBr): cm⁻¹ 3464 (NH), 3362 (NH), 2961 (CH)

| Elemental analysis (%) | |
|---|---|
| Calculated for C₄₀H₅₂N₄S₄ | C: 66.99, H: 7.31, N: 7.81, S: 17.89 |
| Found | C: 66.67, H: 7.21, N: 7.69, S: 17.72 |

### Application Example 1

Into chloroform was dissolved 5,11,17,23-tetra-*tert*-butyl-25,26,27,28-tetraamino-2,8,14,20-tetrasulfonyl[1.9.3.1.1^{3,7}1^{9,13}1^{15,19}]octacosa-1(25),3,5,7(28),9,11,13(27),15,17,19(26),21,23-dodecaene obtained in Example 2 to thereby obtain 4×10⁻⁴ M solution. Thereto was added 4×10⁻⁴ M aqueous acetic acid solution, followed by shaking. The extraction rate after 5 hours was 95%.

### Application Example 2

Into 100 ml of chloroform were dissolved 71.7 mg of 5,11,17,23-tetra-*tert*-butyl-25,26,27,28-tetraamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene and 72.1 mg of 5,11,17,23-tetra-*tert*-butyl-25,26,27,28-tetrahydroxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene. The mixture was shaken together with 100 ml of 3 N hydrochloric acid and then fractionated to a hydrochloric acid phase and a chloroform phase. 5,11,17,23-Tetra-*tert*-butyl-25,26,27,28-tetraamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene was confirmed in the hydrochloric acid phase and chloroform was extracted together with the compound, but 5,11,17,23-tetra-*tert*-butyl-25,26,27,28-tetrahydroxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene was not transferred into the hydrochloric acid phase as an extract.

### Application Example 3

The experiment was carried out in the same manner as in Application Example 2, except that 5,11,17,23-tetra-*tert*-butyl-25,26,27,28-tetraamino-2,8,14,20-tetrathia[1.9.3.1.1^{3,7}1^{9,13}1^{15,19}]octacosa-1(25),3,5,7(28),9,11,13(27),15,17,19(26),21,23-dodecaene was used instead of 5,11,17,23-tetra-*tert*-butyl-25,26,27,28-tetraamino-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene. 5,11,17,23-Tetra-*tert*-butyl-25,26,27,28-tetraamino-2,8,14,20-tetrathia[1.9.3.1.1^{3,7}1^{9,13}1^{15,19}]octacosa-1(25),3,5,7(28),9,11,13(27),15,17,19(26),21,23-dodecaene was confirmed in the hydrochloric acid phase and chloroform was extracted together with the compound, but 5,11,17,23-tetra-*tert*-butyl-25,26,27,28-tetrahydroxy-2,8,14,20-tetrasulfinyl[1.9.3.1.1^{3,7}1^{9,13}1^{15,19}]octacosa-1(25),3,5,7(28),9,11,13(27),15,17,19(26),21,23-dodecaene was not transferred into the hydrochloric acid phase as an extract.

### INDUSTRIAL APPLICABILITY

According to the production process of the present invention, a cyclic aniline sulfide having a basic skeleton, which is a cage molecule, can be efficiently and easily obtained. Moreover, the cyclic aniline sulfide is useful as a sensor which can be used in a wide pH range such as a low pH range, e.g., in an aqueous acidic solution, and also as an analytical reagent, a raw material for metal-organic composite materials, and a metal scavenger.

## Claims

1. A cyclic aniline sulfide represented by formula (1): 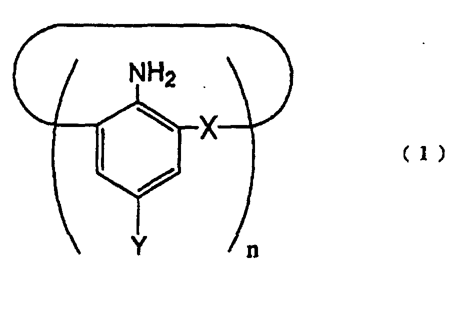 wherein X represents SO or SO₂; Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; plural X's or plural Y's are the same or different; and n represents an integer of 4 to 8.

2. An *N*-hydrocarbon group-substituted cyclic aniline sulfide represented by formula (2): 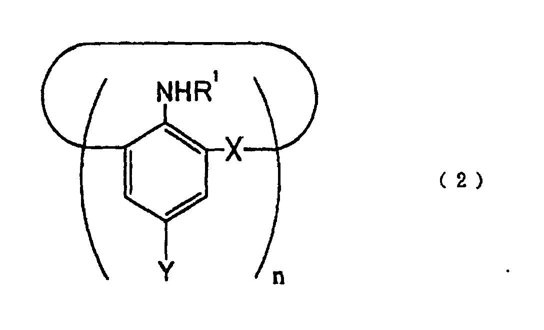 wherein X represents SO or SO₂; Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; R¹ is a hydrocarbon group; plural X's or plural Y's are the same or different; and n represents an integer of 4 to 8.

3. A process for producing an *N*-hydrocarbon group-substituted cyclic aniline sulfide represented by formula (2): 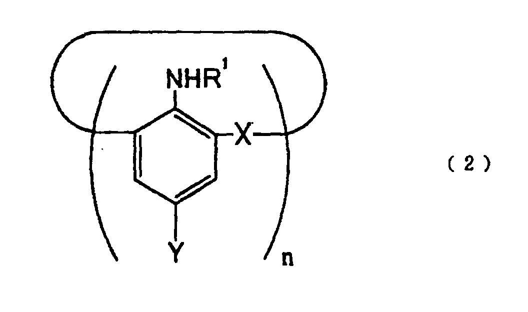 wherein X represents SO or SO₂; Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; R¹ represents a hydrocarbon group; plural X's or plural Y's are the same or different; and n represents an integer of 4 to 8, which comprises reacting a cyclic phenol sulfide represented by formula (3): 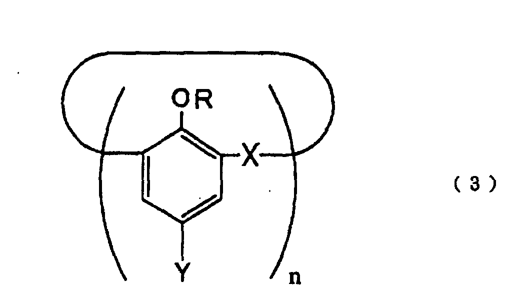 wherein X, Y, and n have the same meanings as described above; and R represents a hydrocarbon group, with a hydrocarbon group-substituted amino-lithium.

4. The process for producing an *N*-hydrocarbon group-substituted cyclic aniline sulfide according to claim 3, wherein the hydrocarbon group-substituted amino-lithium is benzylamino-lithium.

5. A process for producing a cyclic aniline sulfide represented by formula (1): 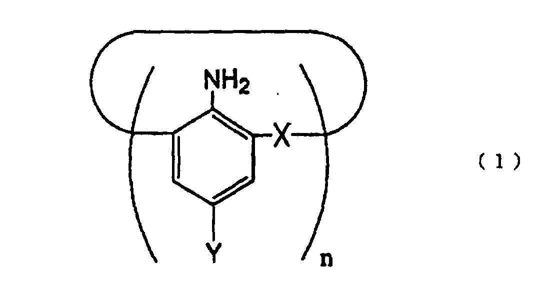 wherein X represents SO or SO₂; Y represents hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; plural X's or plural Y's are the same or different; and n represents an integer of 4 to 8, which comprises debenzylating an *N*-benzyl group-substituted cyclic aniline sulfide represented by formula (2): 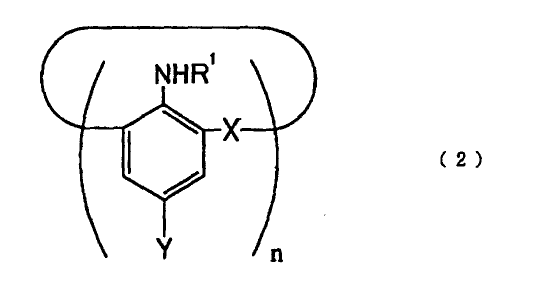 wherein X, Y and n have the same meanings as described above; and R¹ is a hydrocarbon group.

6. The process for producing a cyclic aniline sulfide according to claim 5, wherein the debenzylation is conducted by substitution of a benzyl group with a halogen with a halogenating agent and hydrolysis with a basic catalyst.

7. A cyclic aniline'sulfide represented by formula (4): 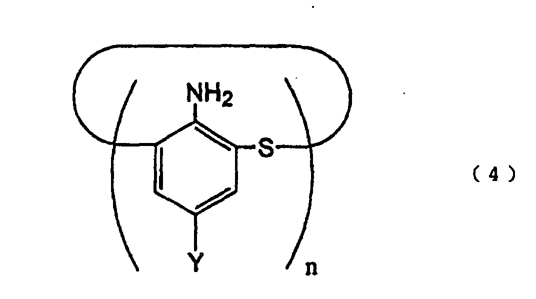 wherein Y represents a hydrogen atom, a hydrocarbon group, a halogenated hydrocarbon group, -COR², -OR³, -COOR⁴, -CN, -CONH₂, -NO₂, -NR⁵R⁶, a halogen atom, -SO₄R⁷ or -SO₃R⁸; R² to R⁸ each represents a hydrogen atom or a hydrocarbon group; plural Y's are be the same or different; and n represents an integer of 4 to 8.

8. A process for producing a cyclic aniline sulfide represented by formula (4): 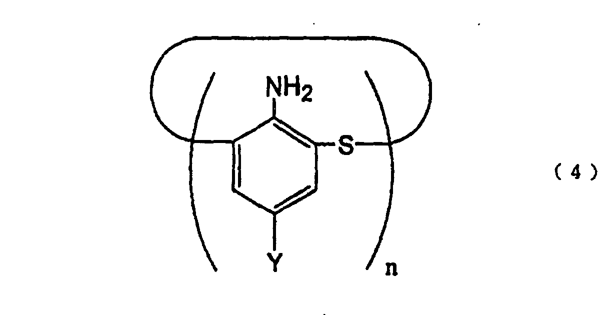 which comprises reducing a cyclic aniline sulfide represented by formula (1): 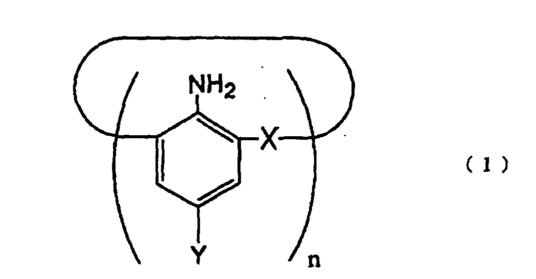 wherein Y and n have the same meanings as described above; X represents SO or SO₂; and plural X's are the same or different.

9. The process for producing a cyclic aniline sulfide according to claim 8, wherein the reduction is conducted in the presence of titanium tetrachloride using lithium aluminum hydride.
